# FASCICULE DE BREVET EUROPEEN

(11) **EP 0 654 482 B1**
(45) Date de publication et mention de la délivrance du brevet: **14.04.1999**
(21) Numéro de dépôt: 94402543.6
(22) Date de dépôt: 10.11.1994
(51) Int. Cl.: C08B 37/00, C08L 5/00, C08L 5/14

(54) **Compositions à base de polymères cationiques et de gomme xanthane anionique**
Zusammensetzungen auf der Basis von kationischen Polymeren und anionischen Xanthangummi
Composition based on cationic polymers and anionic xanthan gum

(30) Priorité: 22.11.1993 US 156189
(43) Date de publication de la demande: 24.05.1995
(73) Titulaire: RHODIA INC., Cranbury, New Jersey 08512 (US)
(72) Inventeur: Yeh, Michael H., Hamilton, New Jersey 08619 (US)
(74) Mandataire: Seugnet, Jean Louis

(56) Documents cités:
- DD-A- 281 966
- FR-A- 2 156 173
- GB-A- 2 064 568
- US-A- 3 236 831

## Description

La présente invention concerne une composition contenant un ou plusieurs polymères cationiques et de la gomme xanthane anionique. La composition contient plus particulièrement des polygalactomannanes à charge cationique en association avec de la gomme xanthane anionique substituée. Les compositions qui en résultent présentent des viscosités supérieures à celles que l'on obtient en mettant en oeuvre séparément les polygalactomannanes et la gomme xanthane ou en utilisant les polygalactomannanes en combinaison avec de la gomme xanthane non ionique.

Les gommes naturelles et synthétiques ont été utilisées comme épaississants pour les produits alimentaires, les revêtements, les peintures, bouillies explosives, les fluides pour puits de pétrole, les cosmétiques et bien d'autres applications fonctionnelles. La gomme xanthane est l'une des gommes naturelles qui a été utilisée sur une grande échelle comme agent de suspension et de viscosité. Les sols aqueux de gomme xanthane sont plastiques par nature et présentent des résistances de gel supérieures aux sols de la plupart des autres gommes. Les mélanges de gomme xanthane avec d'autres gommes sont bien connus et chaque mélange présentent des propriétés uniques qui lui sont propres. Les galactomannanes constituent l'une des familles de gommes que l'on a proposé de mélanger à la gomme xanthane.

A titre d'exemple, Schuppner et al. discutent dans les brevets US 3 507 664, 3 519 434, 3 557 016 et 3 659 026 d'un certain nombre d'utilisations de compositions à base de gomme xanthane et de gomme de caroube. Les utilisations indiquées sont : les gels de lait (brevet US3 507 664), les gels de viande, les gels thermoréversibles d'une manière générale (brevet US 3 557 016) et les applications agricoles brevet US 3 659 026). L'une des idées de base de chacun de ces brevets est que l'association de la gomme xanthane et de la gomme de caroube pour chacune des applications fonctionnelles donne une composition qui, introduite dans un solvant, confère d'excellentes propriétés de gélification.

Le brevet US 4 038 206 suggère que l'on peut obtenir des viscosités plus élevées lorsque la gomme de caroube mise en oeuvre en association avec la gomme xanthane est une gomme de caroube hydroxyalkylée. Les gommes de caroube hydroxyalkylée contiennent des hydroxypropyléthers de la gomme de caroube solubles dans l'eau, des hydroxyéthyléthers de la gomme de caroube et des hydroxybutyléthers de la gomme de caroube. La gomme de caroube hydroxyalkylée est préparée en faisant réagir un oxyde d'alkylène avec la gomme de caroube pour former une ou plusieurs liaisons éther. La quantité molaire d'oxyde d'alkylène par mole de gomme de caroube qui est proposée est comprise entre 0,05 et 0,5.

Le brevet US 4 162 925 suggère d'utiliser des esters phosphatés de gomme de caroube ayant un degré de substitution compris entre environ 0,03 et environ 0,5 en combinaison avec de la gomme xanthane pour former des liquides visqueux et/ou de gels fermes. On suggère plus particulièrement d'utiliser ces produits comme agents de suspension pour les forages des puits de pétrole ainsi que dans les procédés de forage de précision désignés sous le nom de forages à l'aide de fluides. Les exemples du brevet suggèrent que les esters phosphatés utilisées soient de nature anionique.

La combinaison de la gomme xanthane avec d'autres galactomannanes, telles que la gomme de guar et ceux qui dérivent de la gomme de guar fait également l'objet de discussions dans la littérature. Le brevet US 3 748 201, par exemple, propose des compositions épaississantes à base de gomme xanthane et d'hydroxyalkyléthers de gomme de guar. L'hydroxyalkyléther de gomme de guar est obtenu en faisant réagir la gomme de guar avec un oxyde d'alkylène en présence d'un catalyseur alcalin. Le degré de substitution préférentiel de la gomme de guar résultant de la formation de liaisons éther est compris de préférence entre environ 0,2 et environ 1,2. On suggère d'utiliser ces compositions pour teindre des fils tapis et dans les explosifs.

On admet comme hypothèse que l'interaction entre la gomme xanthane et les polygalactomannanes décrits plus haut se fait au niveau de la molécule. Les polygalactomannanes possédant moins de chaînes latérales galactose et/ou une distribution moins uniforme des motifs galactose interagiraient plus fortement avec la gomme xanthane que les polygalactomannanes ayant une teneur plus élevée en galactose et/ou une distribution plus uniforme du galactose sur l'ensemble de la chaîne principale galactose.

Le brevet US 3 467 647 décrit des polysaccharides contenant à la fois des substituants cationiques et anioniques. Parmi les polysaccharides de départ qui sont alors modifiés selon ce brevet, on trouve les amidons, la gomme de caroube et la gomme de guar. Les substituants cationiques comprennent les groupes amino primaires, secondaires ou tertiaires, les groupes ammonium quaternaire, sulfonium ou phosphinium. Les substituants anioniques proposés comprennent les groupes carboxyle, sulfonate, sulfate et phosphate. L'exemple 9 de ce brevet décrit la gomme de guar comme polysaccharide, le triméthylammoniumhydroxypropyle comme groupes cationiques et les phosphates comme groupes anioniques. Le degré de substitution de chacun de ces groupes dans l'exemple en question est de 0,05.

Dans Chem. Abstracts CA115(16): 16250p, la discussion porte sur les utilisations de certaines combinaisons de polymères qui augmentent les viscosités par rapport aux viscosités de chacun des polymères. Les combinaisons mentionnées comprennent le poly(styrènesulfonate) et la gomme xanthane ou bien l'hydroxyéthylcellulose, le poly(vinylsulfonate) et la gomme xanthane, un guar modifié par un sel d'ammonium quaternaire et l'hydroxypropylguar ou l'hydroxyéthylcellulose, et un guar sulfoné et soit une hydroxyéthylcellulose ou une carboxyméthylhydroxyéthylcellulose. On suggère d'utiliser lesdites combinaisons pour la récupération du pétrole.

Le brevet DD 28 19 66 décrit également un agent de gélification contenant des polymères cationiques et anioniques et aboutissant par synergie à une augmentation de la viscosité par rapport aux solutions contenant des quantités séparée de polymères. Le polymère anionique est de préférence un poly(diméthyl-diallylammonium chlorure) contenant des motifs pyrrolidinium, le polymère cationique étant de préférence une carboxyméthylcellulose ayant un degré de substitution compris entre 0,6 et 1,2.

Les brevets US 4 264 322, 4 403 360 et 4 454 617 décrivent des compositions tinctoriales pour fibres textiles. Les compositions contiennent une dispersion de phases gel non miscibles dans lequel la première phase gel est épaissie à l'aide d'un agent de gélification cationique et la seconde qui est dispersée dans la première, à l'aide d'un agent de gélification anionique. Les agents de gélification que l'on suggère d'utiliser pour la première phase comprennent les polygalactomannanes contenant des substituants éther d'ammonium quaternaire. Les agents de gélification que l'on propose de mettre en oeuvre pour la seconde phase comprennent les hydrocolloïdes qui présentent le même type de structure polymère de base que les agents de gélification, la différence étant que le groupe cationique est substitué par un groupe anionique tel que l'acide carboxylique, l'acide sufonique ou un sulfate.

Le brevet DE 1 518 731 indique que l'on peut éthérifier les galactomannanes ou les glucomannanes à l'aide d'un acide β-halogénoéthane sulfonique ou d'acides halogénométhane sulfoniques en présence d'une base afin d'obtenir des compositions pouvant être utilisées comme apprêts textiles, agents d'ensimage et épaississants pour encres d'impression.

Les brevets US 3 912 713 et FR 2 242 401 décrivent des dérivés de la gomme de guar et leurs procédés de préparation. Les dérivés sont obtenus par addition d'un substituant aux écailles de gomme de guar en présence d'eau et, d'une manière typique, d'une base. Parmi les substituants (agents de dérivatisation) que l'on suggère d'utiliser dans ces brevets on trouve les acides halogénoalkylsulfoniques, tels que les acides bromoéthanesulfonique et chlorohydroxypropanesulfonique, les acides époxyalkyl sulfoniques, tel que l'acide époxypropane sulfonique, et les acides alpha, bêta-alkylène sulfoniques, tel que l'acide éthylène sulfonique. On propose d'utiliser lesdits composés comme agents épaississants, agents de tension, d'ensimage et apprêts, comme colloïdes de protection et comme agents de stabilisation de dispersions et émulsions.

Le brevet US 4 031 305 décrit des sulfohydroxypropyléthers de polygalactomannanes ayant un degré de substitution compris entre environ 0,01 et 3. Les éthers sont obtenus en mettant en contact de la gomme de guar ou de la gomme de caroube solides avec un acide 3-halogéno-2-hydroxypropanesulfonique ou un sel d'acide en présence d'une base. On suppose que les éthers de galactomannanes sont anioniques par nature et on propose de les utiliser dans les industries du pétrole, du textile, de l'imprimerie, du papier, de l'alimentation et de la pharmacie.

Le brevet US 4 057 509 décrit la formation d'un gel acide par la mise en contact d'un polygalactomannane avec un halogénure d'allyle, le polygalactomannane allyl éther ainsi obtenu étant soumis ensuite à un courant d'anhydride sulfureux. On propose d'utiliser ces gels dans les compositions pour boues de forage de puits de pétrole et dans les compositions de fracturation pour puits de pétrole.

En dépit de tout ce qui précède, il existe toujours un besoin de compositions présentant des viscosités plus élevées et reposant, en plus des forces moléculaires, sur d'autres forces.

Selon la présente invention, on met à disposition une nouvelle combinaison qui présente une viscosité supérieure à celle de chacun des polymères de départ et fait appel à la fois à des forces ioniques et moléculaires. La nouvelle combinaison contient de la gomme xanthane substituée anionique et un ou plusieurs polymères cationiques.

L'un des modes de réalisation de la présente invention concerne une composition constituée d'un mélange d'environ 1 à environ 99 parties d'un ou de plusieurs polymères cationiques, de préférence, un polygalactomannane ayant un degré de substitution de l'ordre de 0,01 à 3,00, et d'environ 1 à environ 99 parties de gomme xanthane substituée anionique.

Dans les modes de réalisation que l'on préfère plus particulièrement, le polymère cationique est un polygalactomannane ayant un degré de substitution compris entre environ 0,01 et environ 1,00, le caractère anionique du polymère xanthane étant obtenu par substitution sur le squelette xanthane de dérivés des monomères à insaturation éthylénique qui contiennent des groupes sulfonate anionique.

Ces mélanges sont particulièrement efficaces comme épaississants. On peut les utiliser dans un grand nombre d'applications fonctionnelles, à savoir les produits alimentaires, les explosifs, produits chimiques pour champs de pétrole, les applications agricoles, les cosmétiques et autres similaires.

Un autre mode de réalisation de la présente invention concerne un procédé de fabrication d'un liquide visqueux ou d'un gel. Le procédé comporte une phase dans laquelle on ajoute au solvant, de préférence de l'eau, pour 100 parties de liquide visqueux ou de gel, de 0,1 à environ 2,0 parties d'une composition constituée d'un mélange dont le rapport est d'environ 1 à environ 99 parties d'un ou de plusieurs polymères cationiques et d'environ 1 à environ 99 parties de gomme xanthane substituée anionique.

En conséquence, la présente invention a pour objet de mettre à disposition une nouvelle composition constituée d'un mélange comportant des matières qui, lorsqu'elles sont combinées, présentent un profil de viscosité supérieur à celui de matières prises individuellement ou de matières combinées non chargées.

La présente invention a pour objet également de mettre à disposition un procédé de production d'un liquide visqueux ou gel en mettant en oeuvre une nouvelle composition constituée d'un mélange.

Un autre objet de l'invention est de mettre à disposition un produit alimentaire, un explosif, un produit chimique pour champs de pétrole, une fibre textile ou un produit cosmétique contenant la nouvelle composition constituée d'un mélange.

Pour l'homme du métier, lesdits objets et autres seront évidents s'il se réfère à la description détaillée du mode de réalisation préférentiel.

Dans la description du mode de réalisation préférentiel, on utilise une terminologie particulière pour être plus clairs. Cette terminologie vise à mieux cerner le mode de réalisation décrit ainsi que tous les équivalents techniques qui fonctionnent d'une manière similaire dans un but similaire pour obtenir un résultat similaire.

La présente invention comprend une composition constituée d'un mélange à base de polysaccharide amphotère contenant environ de 1 à 99 parties d'un ou de plusieurs polymères cationiques, de préférence, des polygalactomannanes ayant un degré de substitution d'environ 0,01 à environ 3,00, et d'environ 99 à environ 1 parties de gomme xanthane substituée anionique. Dans la pratique, les matières peuvent être mélangées à l'état sec ou, de préférence, chacune est dispersée dans un fluide, de préférence l'eau, et, ensuite, on mélange les fluides.

Le premier élément du mélange contient un ou plusieurs polymères cationiques, de préférence des polygalactomannanes ayant un degré de substitution compris entre environ 0,01 et environ 3,0. On préfère plus particulièrement les polygalactomannanes ayant un degré de substitution compris entre environ 0,05 et environ 2,0, le degré de substituion que l'on préfère le plus étant compris entre environ 0,1 et environ 1,0.

Les polygalactomannanes sont des polysaccharides constitués principalement de motifs galactose et mannose on les trouve généralement dans l'endosperme des graines de légumineuses, tels que le guar, le caroube, le gléditschia (acacia à trois épines), le flamboyant (flame tree). La farine de guar, par exemple, est composée en majeure partie d'un galactomannane qui est essentiellement une chaîne linéaire mannane comportant des branches à motif unique de galactose. Les motifs mannose sont liés à l'intérieur d'une liaison 1-4-β-glycosidique et le galactose vient se brancher par l'intermédiaire d'une liaison 1-6 sur les motifs mannose alternés. C'est la raison pour laquelle le rapport du galactose au mannose dans le polymère du guar est de un à deux. Le poids moléculaire de la gomme de guar est d'environ 1 500 000.

La gomme de caroube est également une gomme de polygalactomannane de structure similaire dans laquelle le rapport du galactose au mannose est de un à quatre. Les gommes de guar et de caroube constituent les sources préférentielles de polygalactomannanes, essentiellement parce qu'on les trouve dans le commerce.

Dans la pratique, le polygalactomannane peut est mis en oeuvre soit à l'état naturel (c'est-à-dire gommes de guar ou de caroube pures) ou sous la forme de dérivés. Les dérivés de polygalactomannanes contiennent un ou plusieurs groupes non ioniques.

Comme exemples de ces polygalactomannanes on citera l'hydroxypropyl guar, I'hydroxyéthyl guar et autres similaires. Ces dérivés des polygalactomannanes sont commercialisés par Rhône-Poulenc Inc. sous les marques Jaguar 8012, Jaguar 8060, Jaguar 8000, Jaguar HP-20 et Jaguar HP-23.

Par le terme "degré de substitution" tel qu'il est employé ici, on entend la substitution moyenne des groupes cationiques ou anioniques par motif de sucre anhydro dans les gommes de polygalactomannanes. Dans la gomme de guar, I'unité de base du polymère est constituée par deux motifs mannone comportant une liaison glycosidique et par une unité galactose fixée sur le groupe hydroxyle de l'un des motifs mannose. En moyenne, chacun des motifs sucre anhydro comporte trois sites hydroxyle disponibles. Un degré de substitution de trois signifie que la totalité des sites hydroxyle disponibles a été estérifiée avec des groupes formate ester.

Il existe d'autres matières que l'on peut choisir comme polymère de départ avant substitution par un ou plusieurs groupes cationiques, à savoir les amidons, celluloses et la gomme xanthane. Comme exemples d'amidons, on citera les amidons naturels et modifiés, tels que les amidons dextrinés, hydrolysés, oxydés, réticulés, alkylés, hydroxyalkylés, acétylés ou fractionnés (par exemple, l'amylose et l'amylopectine). On peut utiliser des amidons de n'importe quelle origine, par exemple, de l'amidon de maïs, de blé, de pomme de terre, de tapioca, de sago, de riz, de l'amidon de maïs cireux ou de l'amidon de maïs fortement amylosé.

Comme exemples de celluloses, on citera l'hydroxyéthylcellulose, l'hydroxypropylcellulose et les alkylcelluloses.

A titre d'exemples, les groupes cationiques qui conviennent pour la mise en oeuvre de la présente invention comprennent les groupes ammonium quaternaire. Les groupes ammonium quaternaire typiques sont le chlorure et le bromure de tétraméthylammonium, le chlorure et le bromure de benzyltriméthylammonium, le chlorure et le bromure de tétraéthylammonium, le chlorure et le bromure de tétrabutylammonium, le chlorure et le bromure de méthylpyridinium, le chlorure et le bromure de benzylpyridinium, le chlorure et le bromure de triméthyl-p-chlorobenzylammonium, etc. dans lesquels chacun desdits groupes donne un dérivé sous la forme d'un radical qui est substitué dans un agent de gélification hydrocolloïdal au moyen d'une liaison alkylène ou oxyalkylène. La structure polymère des polygalactomannanes appropriés contenant des groupes cationiques comprend les polymères et copolymères vinyliques, les résines échangeuses d'ions, les polysaccharides et autres similaires. Commes exemples de cette classe d'hydrocolloïdes on citera les gommes de polygalactomannane contenant des substituants éther d'ammonium quaternaire, tels que ceux qui sont décrits dans le brevet US 4 031 307 : 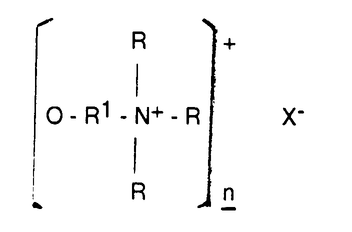 dans lesquels R représente un groupe alkyle contenant de un à environ six atomes de carbone, R1 est un groupe alkylène contenant de un à environ six atomes de carbone, X est le chlore ou le brome et n est un nombre entier correspondant au degré de substitution des substituants éther d'ammonium quaternaire dans un agent gélifiant cationique à base de gomme de polygalactomannane. Lesdits groupes alkyle et alkylène peuvent contenir d'autres atomes, tels que des atomes d'oxygène, de soufre et d'halogène. Les dérivés cationiques de la gomme de guar ou de caroube ont obtenus en mettant en contact de la gomme de guar ou de caroube solide avec un composé d'ammonium quaternaire substitué par un halogénoalkyle et un excès stochiométrique d'hydroxyde de métal alcalin ou d'hydroxyde d'ammonium dans un milieu réactionnel contenant une solution aqueuse de solvant hydrosoluble, à une température comprise entre environ 10°C et environ 100°C pendant une durée suffisante pour obtenir un degré de substitution par les groupes éther d'ammonium quaternaire compris entre environ 0,01 et environ 0,40. La gomme de guar solide ou tout autre polygalactomannane éthérifié peut se présenter sous la forme d'écailles d'endosperme ou sous la forme de poudre finement divisée obtenue à partir des paillettes d'endosperme. Il est important que la gomme de polygalactomannane éthérifiée avec des groupes ammonium quaternaire reste en phase solide dans le milieu réactionnel pendant la durée de la réaction.

Le brevet US 4 031 307 donne de plus amples détails sur la synthèse de ces polymères. Ce brevet est incorporé par référence dans la mesure où cela est nécessaire.

Comme exemples de polygalactomannanes commercialisés, contenant un ou plusieurs groupes ammonium quaternaire cationiques substitués, on citera les Jaguar C-13, Jaguar C-13S, Jaguar C-14, Jaguar C-17 et Jaguar C-14S, tous commercialisés par Rhône-Poulenc Inc.

Les autres polymères cationiques comprennent ceux qui contiennent d'autres groupes cationiques, tels que les sels d'acide d'amines primaires, secondaires, les groupes sulfonium ou phosphonium.

L'autre composant de la composition constituée par un mélange selon l'invention comprend la gomme xanthane qui contient des charges anioniques, de préférence par substitution de groupes anioniques sur le squelette moléculaire du xanthane. La gomme xanthane est obtenue en règle générale à partir du produit de fermentation issu de l'action de a bactérie **Xanthomonas campestris** sur les hydrates de carbone. On considère que les autres espèces de Xanthomonas entrent dans le cadre de la présente invention. Les brevets US 4 041 234 et 4 229 825 décrivent la production de gomme xanthane à partir d'un bouillon de fermentation. Dans la mesure où cela est nécessaire, les documents cités ci-dessus sont font partie du présent brevet par référence. La gomme xanthane obtenue à partir du bouillon de fermentation est, en règle générale, séparée du bouillon, puis lavée et séchée. Les particules de gomme xanthane sont normalement anionique par nature. Comme exemples non limitatifs de particules de gomme xanthane appropriée que l'on peut mettre en oeuvre selon la présente invention, on citera les produits vendus par Rhône-Poulenc Inc. sous les marques Rhiodigel, Rhodopol 23P et Rhodopol 23.

La nature anionique du polymère xanthane est obtenu de préférence par la substitution d'un ou de plusieurs groupes sulfonate sur le squelette xanthane. On préfère plus particulièrement comme substituants anioniques les dérivés des monomères à insaturation éthylénique qui contiennent un ou plusieur groupes sulfonate. Comme exemples de ces monomères, on citera le sel de sodium de l'acide 2-acrylamido-2-méthylpropane sulfonique et le sel de sodium de l'acide 1-allyloxy-2-hydroxypropylsulfonique. Le premier monomère dérive de l'acide 2-acrylamido-2-méthylpropane sulfonique commercialisé par Lubrizol sous la marque LZ 2401, le deuxième est commercialisé par Rhône-Poulenc Inc. sous la marque Sipomer Cops I.

Il existe d'autres groupes anioniques que l'on peut substituer sur le squelette xanthane, à savoir les groupes sulfate, carboxyle ou phosphate.

La formation des liaisons éther entre le substituant sulfonaté et le polymère xanthane se fait en ajoutant directement le substituant à la gomme xanthane, de préférence, en présence d'un solvant, tel que le toluène. La température réactionnelle est généralement comprise entre environ 10°C et environ 100°C. On peut favoriser la réactivité du polymère vis-à-vis du substituant en mettant en oeuvre une petite quantité d'initiateur. Comme exemples d'initiateurs appropriés, on citera le persulfate d'ammonium. On peut ajouter aussi des tampons pH, tel que le disodium phosphate, qui auront un effet optimum. Après le mélange ou tout autre association, le rapport du polymère cationique à la gomme xanthane anionique dans la présente invention peut varier dans de larges limites, par exemple, entre environ 1 - environ 99 % en poids de polymère cationique pour environ 99 - 1 % en poids de gomme xanthane anionique, le total étant de 100 parties en poids. Le rapport préférentiel est est d'environ 5 - 95 parties en poids de polymère cationique pour environ 95 - 5 parties en poids de polymère anionique, le total étant de 100 parties en poids.

Dans la pratique, les compositions selon l'invention peuvent servir utilement d'épaississants lorsqu'elles sont ajoutées à un solvant, habituellement l'eau. Généralement, on ajoute environ 0,1 - 0,2 parties de polymère xanthane anionique et de polymère cationique pour 100 parties de liquide visqueux ou de gel.

Les compositions constituées de mélanges à base de polysaccharide amphotère sont obtenues habituellement en mélangeant des solutions de polymères cationiques et anioniques en quantités respectives telles que les charges positives et négatives sont équilibrées de part et d'autre. Les quantités respectives de solutions anioniques et cationiques sont ajoutées les unes aux autres, essentiellement sur la base du degré de substitution de chacune. Par exemple, on peut ajouter de plus grandes quantités d'un polymère cationique à faible degré de substitution à des quantités plus faibles de solution anionique ayant un degré important de substitution.

Si, dans le mode de réalisation préférentiel, les solutions anioniques et cationiques sont ajoutées en quantités relatives pour obtenir une solution à charge neutre, on peut faire varier les quantités de chacune pour obtenir des solutions ayant une charge globale positive ou négative. Bien que l'augmentation de la viscosité ne soit pas aussi importante que lorsque l'on prépare une solution à charge neutre, on peut obtenir une augmentation de la viscosité par synergie en procédant à des additions dont les charges ne sont pas équilibrées.

Au lieu de mélanger des solutions aqueuses séparées de polymères cationiques et anioniques pour obtenir le mélange amphotère selon l'invention, on peut mélanger le polymère cationique et la gomme xanthane anionique à l'état sec dans le rapport voulu et ajouter ensuite à l'eau comme indiqué plus haut. Pour faciliter les manipulations et la dispersion, les gommes devraient avoir une granulométrie inférieure à environ 100 mailles (mesh). D'autres composants, tels que charges, mouillants, dispersants, bactéricides, fongicides et autres similaires peuvent, s'il le faut, être mélangés aux mélanges en poudre selon l'invention

Il est connu que l'interaction entre différents polygalactomannanes peut aboutir à renforcer les viscosités. Ce qui est surprenant en ce qui concerne la présente invention, c'est que lorsque l'on met en oeuvre des polymères cationiques en association avec de la gomme xanthane anionique on obtient un double avantage. Le premier avantage réside dans l'augmentation de la viscosité provoquée par l'interaction moléculaire entre le polymère cationique et le xanthane anionique. Le deuxième avantage est constitué par l'interaction ionique qu'implique la mise en oeuvre d'un polymère cationique avec la gomme xanthane anionique. En mettant en oeuvre à la fois les forces moléculaires et les forces ioniques, on obtient une véritable amélioration par rapport aux mélanges à base de polysaccharides non ioniques.

En raison de leurs propriétés épaississantes, les applications possibles des nouvelles compositions sont très nombreuses. On peut les utiliser entre autres comme agents de suspension de différents solides, tels que les produits chimiques pour l'industrie du pétrole, dans la teinture des fibres textiles, dans les produits alimentaires, les cosmétiques et les produits de soins corporels, dans les produits pour l'agriculture, dans les explosifs, la fabrication du papier, etc. L'homme du métier appréciera volontiers d'autres applications.

L'invention fait l'objet d'une description beaucoup plus détaillée dans les exemples suivants qui ne sont pas limitatifs :

### EXEMPLE 1

On ajoute une solution de 2,4 parties de persulfate d'ammonium et de 3 parties de disodium phosphate dans 50 parties d'eau à 322 parties de Rhodigel qui est une gomme xanthane fabriquée par Rhône-Poulenc Inc. dans 1 300 parties de toluène et on chauffe la solution à 70°C. On ajoute 50 parties de solution de sodium-2-acrylamido-2-méthylpropane sulfonate monomère et on maintient la température réactionnelle entre 65 et 70°C pendant 2 heures. On refroidit le mélange, on le filtre et on le déshydrate avec du méthanol. Le rendement est de 370,8 parties, la teneur en humidité étant de 12 %.

### RESULTATS DES TESTS EXPERIMENTAUX

On utilise les échantillons suivants pour comparer les viscosités de solutins aqueuses à 1 % à 25°C, deux heures après hydratation, dans différentes conditions de cisaillement :
Echantillon A - Rhodigel (100 %) (gomme xanthane)
Echantillon B - Composition selon l'exemple 1 (100 %)
Echantillon C - Jaguar C-14 (100 %) (guar cationique)
Echantillon D - Jaguar C-17 (100 %) (guar cationique)
Echantillon E - Mélange 50 : 50 en poids d'Echantillon A et d'Echantillon C
Echantillon F - Mélange 50 : 50 en poids d'Echantillon B et d'Echantillon C
Echantillon G - Mélange 50 : 50 en poids d'Echantillon A et d'Echantillon D
Echantillon H - Mélange 50 : 50 en poids d'Echantillon B et d'Echantillon D

Les taux de cisaillement, exprimés en t/mn, et les viscosités, exprimées en centipoises, figurent dans le Tableau 1.

**TABLEAU 1**

| T/mn | 0,5 | 1 | 2,5 | 5 | 10 | 20 | 50 |
|---|---|---|---|---|---|---|---|
| Echantillon A | 50 000 | 28 500 | 13 200 | 7 600 | 4 200 | 2 400 | 1 100 |
| Echantillon B | 68 000 | 34 000 | 17 200 | 9 200 | 5 100 | 2 800 | 1 280 |
| Echantillon C | 32 000 | 26 000 | 18 400 | 12 800 | 8 600 | 5 500 | 2 820 |
| Echantillon D | 10 000 | 9 000 | 6 800 | 5 200 | 3 800 | 2 650 | 1 500 |
| Echantillon E | 57 000 | 31 500 | 14 000 | 7 800 | 4 400 | 2 500 | 1 400 |
| Echantillon F | 68 000 | 36 000 | 16 000 | 9 200 | 5 000 | 3 300 | 2 000 |
| Echantillon G | 148 000 | 84 000 | 36 800 | 20 000 | 10 800 | 6 200 | 2 600 |
| Echantillon H | 152 000 | 80 000 | 39 600 | 20 800 | 12 000 | 8 000 | 3 950 |

Si l'on examine les données figurant dans le Tableau 1, on remarque que l'Echantillon F qui est un mélange de guar cationique et de gomme xanthane anionique présente pour tous les taux de cisaillement une viscosité supérieure à celle de l'Echantillon E qui est un mélange de guar cationique et de gomme xanthane non substituée. Bien que moins prononcée, l'Echantillon H qui est un mélange d'un guar cationique différent et de gomme xanthane anionique présente, notamment pour les taux de cisaillement plus élevés, une viscosité supérieure à celle de l'Echantillon G qui est un mélange de guar cationique et de gomme xanthane non substituée. De plus, l'Echantillon B qui contient de la gomme xanthane substituée anionique présente des viscosités supérieures à celles de l'Echantillon A qui ne comporte de substitution anionique.

Après avoir donné une description détaillée de l'invention et si l'on se réfère aux modes de réalisation préférentiels de cette invention, il apparaît qu'il est possible de procéder à des modifications et variations sans sortir du champ des revendications ci-après.

## Revendications

1. Composition caractérisée en ce qu'elle contient un mélange d'environ 1 - 99 parties en poids d'un ou de plusieurs polymères cationiques, et d'environ 99 - 1 parties en poids de gomme xanthane anionique substituée, le total du ou des polymères cationiques et de la gomme xanthane anionique substituée étant de 100 parties en poids.

2. Composition selon la revendication 1, caractérisée en ce que le polymère cationique est un ou plusieurs polygalactomannanes ayant un degré de substitution compris entre 0,01 à 3.

3. Composition selon la revendication 2, caractérisée en ce que le ou les polygalactomannanes sont des dérivés cationiques de la gomme de guar ou de la gomme de caroube.

4. Composition selon la revendication précédente, caractérisée en ce que le ou les polygalactomannanes sont des dérivés cationiques de la gomme de guar.

5. Composition selon l'une quelconque des revendications, caractérisée en ce que le ou les polygalactomannanes sont substitués par un ou plusieurs groupes cationiques choisis parmi les groupes d'ammonium quatemaire, de sels d'acides d'amines primaires, secondaires ou tertiaires, du sulfonium, du phosphonium, et leurs mélanges.

6. Composition selon l'une quelconque des revendications précédentes, caractérisée en ce que la gomme xanthane anionique substituée résulte de la substitution sur son squelette moléculaire d'un ou de plusieurs groupes anioniques choisis parmi les sulfonates, les sulfates, les carboxyles, et les phosphates.

7. Composition selon la revendication 6, caractérisée en ce que la gomme xanthane est substituée sur son squelette par des dérivés des monomères à insaturation éthylénique qui contiennent un ou plusieurs groupes sulfonates.

8. Composition selon l'une quelconque des revendications 1 à 7, caractérisée en ce qu'elle contient un mélange d'un polygalactomannane ayant un degré de substitution compris entre 0,01 et 1,00 et d'une gomme xanthane anionique, substituée sur son squelette par des dérivés des monomères à insaturation éthylénique qui contiennent des groupes sulfonates.

9. Composition selon l'une des revendications 7 et 8, caractérisée en ce que les monomères à insaturation éthylénique sont choisis dans le groupe comprenant l'acide 2-acrylamido-2-méthylpropane sulfonique, et l'acide 1-allyloxy-2-hydroxypropyl sulfonique et leurs sels.

10. Composition selon l'une des revendications précédentes, caractérisée en ce que la quantité et le degré de substitution du ou des polymères cationiques et de la gomme xanthane anionique substituée sont proportionnés de façon à ce que l'on obtienne une composition contenant un mélange à charge neutre.

11. Composition selon l'une des revendications 1 à 10, caractérisée en ce que le ou les polygalactomannanes cationiques et la gomme xanthane anionique substituée peuvent être rnélangés à l'état sec dans le rapport voulu, et ensuite ajoutés à l'eau.

12. Composition selon l'une quelconque des revendications 1 à 10, caractérisée en ce que le ou les polygalactomannanes cationiques et la gomme xanthane anionique substituée sont mis chacun séparément en suspension dans un solvant ou fluide, en vue de former des solutions qui, ensuite, sont mélangées.

13. Composition selon la revendication 12, caractérisée en ce que le ou les polygalactomannanes cationiques et la gomme xanthane anionique substituée sont dispersés dans un solvant ou fluide. puis ajoutés les uns aux autres en quantités relatives pour obtenir une solution à charge neutre.

14. Composition selon l'une quelconque des revendications 12 ou 13, caractérisée en ce que le solvant ou fluide, est de l'eau.

15. Utilisation d'une composition selon l'une quelconque des revendications précédentes pour la fabrication d'un liquide visqueux ou d'un gel, caractérisée en ce que pour obtenir 100 parties en poids de liquide visqueux ou de gel, on ajoute au solvant, 0,1 à 2,0 parties en poids d'une composition telle que définie précédemment.

16. Utilisation d'une composition selon la revendication précédente pour la fabrication d'un liquide visqueux ou d'un gel, caractérisée en ce que le solvant est de l'eau.

17. Utilisation d'une composition selon l'une quelconque des revendications 1 à 14, ou d'un liquide visqueux ou d'un gel fabriqué selon l'une des revendications 15 et 16, comme épaississants dans les produits alimentaires, les explosifs, les produits chimiques pour champs de pétrole, les applications agricoles, les produits cosmétiques, les fibres textiles, les produits pour soins corporels, et la fabrication du papier.

## Claims

1. Composition, characterized in that it comprises a mixture of approximately 1-99 parts by weight of one or more cationic polymers and of approximately 99-1 parts by weight of substituted anionic xanthan gum, the total of the cationic polymer or polymers and of the substituted anionic xanthan gum being 100 parts by weight.

2. Composition according to claim 1, characterized in that the cationic polymer is one or more polygalactomannans having a degree of substitution of between 0.01 and 3.

3. Composition according to claim 2, characterized in that the polygalactomannan or polygalactomannans are cationic derivatives of guar gum or of locust bean gum.

4. Composition according to the preceding claim, characterized in that the polygalactomannan or polygalactomannans are cationic derivatives of guar gum.

5. Composition according to any one of the preceding claims, characterized in that the polygalactomannan or polygalactomannans are substituted by one or more cationic groups chosen from the following groups: quaternary ammonium, acids salts of primary, secondary or tertiary amines, sulphonium, phosphonium and their mixtures.

6. Composition according to any one of the preceding claims, characterized in that the substituted anionic xanthan gum results from the substitution on its molecular skeleton of one or more anionic groups chosen from sulphonates, sulphates, carboxylates and phosphates.

7. Composition according to claim 6, characterized in that the xanthan gum is substituted on its skeleton by derivatives of ethylenically unsaturated monomers which comprise one or more sulphonate groups.

8. Composition according to any one of claims 1 to 7, characterized in that it comprises a mixture of a polygalactomannan having a degree of substitution of between 0.01 and 1.00 and of an anionic xanthan gum, the gum being substituted on its skeleton by derivatives of ethylenically unsaturated monomers which comprise sulphonate groups.

9. Composition according to either of claims 7 and 8, characterized in that the ethylenically unsaturated monomers are chosen from the group comprising 2-acrylamido-2-methylpropanesulphonic acid and 1-allyloxy-2-hydroxypropanesulphonic acid and their salts.

10. Composition according to one of the preceding claims, characterized in that the amount and the degree of substitution of the cationic polymer or polymers and of the substituted anionic xanthan gum are proportioned so that a composition comprising a neutrally charged mixture is obtained.

11. Composition according to one of claims 1 to 8, characterized in that the cationic polygalactomannan or polygalactomannans and the substituted anionic xanthan gum can be mixed in the dry state in the desired ratio and subsequently added to water;

12. Composition according to any one of claims 1 to 10, characterized in that the cationic polygalactomannan or polygalactomannans and the substituted anionic xanthan gum are each suspended separately in a solvent or fluid, for the purpose of forming solutions which are subsequently mixed.

13. Composition according to claim 12, characterized in that the cationic polygalactomannan or polygalactomanns and the substituted anionic xanthan gum are dispersed in a solvent or fluid, and then added to one another in relative amounts in order to obtain a neutrally charged solution.

14. Composition according to one of claims 12 or 13, characterized in that the solvent or fluid, is water.

15. Use of a composition according to any one of the preceding claims for the manufacture of a viscous liquid or of a gel, characterized in that, in order to obtain 100 parts by weight of viscous liquid or of gel, 0.1 to 2.0 parts by weight of a composition as defined above is/are added to the solvent.

16. Use of a composition according to the preceding claim for the manufacture of a viscous liquid or of a gel, characterized in that the solvent is water.

17. Use of a composition according to any one of claims 1 to 14 or of a viscous liquid or of a gel manufactured according to either of claims 15 and 16 as thickeners in foodstuffs, explosives, chemicals for oil fields, agricultural applications, cosmetic products, textile fibres, body care products and the manufacture of paper.

## Patentansprüche

1. Zusammensetzung, dadurch gekennzeichnet, daß sie eine Mischung von ungefähr 1-99 Gewichtsteilen eines oder mehrerer kationischer Polymere und ungefähr 99-1 Gewichtsteilen von substituiertem anionischem Xanthangummi enthält, wobei die Gesamtmenge von dem oder den kationischen Polymeren und dem substituierten anionischen Xanthangummi 100 Gewichtsteile beträgt.

2. Zusammensetzung nach Anspruch 1, dadurch gekennzeichnet, daß das kationische Polymer ein oder mehrere Polygalaktomannane mit einem Substitutionsgrad zwischen 0,01 und 3 ist.

3. Zusammensetzung nach Anspruch 2, dadurch gekennzeichnet, daß das oder die Polygalaktomannane kationische Derivate von Guar Gum oder von Johannisbrotgummi ist.

4. Zusammensetzung nach dem vorhergehenden Anspruch, dadurch gekennzeichnet, daß das oder die Polygalaktomannane kationische Derivate von Guar Gum sind.

5. Zusammensetzung nach einem der vorhergehenden Ansprüche, dadurch gekennzeichnet, daß das oder die Polygalaktomannane durch eine oder mehrere kationische Gruppen substituiert sind, ausgewählt aus den Gruppen von quaternärem Ammonium, Salzen von Säuren und primären, sekundären oder tertiären Aminen, Sulfonium, Phosphonium und Mischungen davon.

6. Zusammensetzung nach einem der vorhergehenden Ansprüche, dadurch gekennzeichnet, daß der substituierte anionische Xanthangummi das Ergebnis einer Substitution von einer oder mehreren anionischen Gruppen, ausgewählt aus den Sulfonaten, Sulfaten, Carboxylen und Phosphaten, in seinem Molekülgerüst ist.

7. Zusammensetzung nach Anspruch 6, dadurch gekennzeichnet, daß der Xanthangummi an seinem Gerüst durch Derivate von ethylenisch ungesättigten Monomeren substituiert ist, die eine oder mehrere Sulfonatgruppen enthalten.

8. Zusammensetzung nach einem der Ansprüche 1 bis 7, dadurch gekennzeichnet, daß sie eine Mischung von einem Polygalaktomannan mit einem Substitutionsgrad zwischen 0,01 und 1,00 und einem anionischen Xanthangummi enthält, der an seinem Gerüst durch Derivate von ethylenisch ungesättigten Monomeren substituiert ist, die Sulfonatgruppen enthalten.

9. Zusammensetzung nach einem der Ansprüche 7 oder 8, dadurch gekennzeichnet, daß die ethylenisch ungesättigten Monomere aus der Gruppe ausgewählt sind, umfassend 2-Acrylamido-2-methylpropansulfonsäure und 1-Allyloxy-2-hydroxypropylsulfonsäure und deren beider Salze.

10. Zusammensetzung nach einem der vorhergehenden Ansprüche, dadurch gekennzeichnet, daß die Menge und der Substitutionsgrad des oder der kationischen Polymere und des substituierten anionischen Xanthangummis so bemessen sind, daß man eine Zusammensetzung, die eine Mischung neutraler Ladung enthält, erzielt.

11. Zusammensetzung nach einem der Ansprüche 1 bis 10, dadurch gekennzeichnet, daß das oder die kationischen Polygalaktomannane und der substituierte anionischen Xanthangummi im trockenen Zustand in dem gewünschten Verhältnis gemischt werden können, und anschließend zu Wasser hinzugefügt werden.

12. Zusammensetzung nach einem der Ansprüche 1 bis 10, dadurch gekennzeichnet, daß das oder die kationischen Polygalaktomannane und der substituierte anionische Xanthangummi jeweils getrennt in einem Lösemittel oder Fluid, suspendiert werden, um Lösungen zu bilden, die nachfolgend gemischt werden.

13. Zusammensetzung nach Anspruch 12, dadurch gekennzeichnet, daß das oder die kationischen Polygalaktommane und der substituierte anionische Xanthangummi in einem Lösemittel oder Fluid, dispergiert werden, und danach das eine dem anderen in solchen Verhältnismengen zugegeben wird, daß eine Lösung neutraler Ladung erhalten wird.

14. Zusammensetzung nach einem der Ansprüche 12 oder 13, dadurch gekennzeichnet, daß das Lösemittel oder Fluid, Wasser ist.

15. Verwendung einer Zusammensetzung nach einem der vorhergehenden Ansprüche zur Herstellung einer viskosen Flüssigkeit oder eines Gels, dadurch gekennzeichnet, daß man zu dem Lösemittel 0,1 bis 2,0 Gewichtsteile einer Zusammensetzung, wie oben definiert, zufügt, um 100 Gewichtsteile viskoser Flüssigkeit oder eines Gels zu erhalten.

16. Verwendung einer Zusammensetzung nach dem vorhergehenden Anspruch zur Herstellung einer viskosen Flüssigkeit oder eines Gels, dadurch gekennzeichnet, daß das Lösemittel Wasser ist.

17. Verwendung einer Zusammensetzung gemäß einen der Ansprüche 1 bis 14 oder einer viskosen Flüssigkeit oder eines Gels, die/das gemäß einem der Ansprüche 15 oder 16 hergestellt wurde, als Verdickungsmittel bei Nahrungsmitteln, Sprengstoffen, chemischen Erzeugnissen für Ölfelder, landwirtschaftliche Anwendungen, kosmetischen Erzeugnissen, Textilfasern, Körperpflegemitteln und bei der Papierherstellung.
